# EUROPEAN PATENT SPECIFICATION

(11) **EP 3 296 401 B1**
(45) Date of publication and mention of the grant of the patent: **19.02.2020**
(21) Application number: 16189598.2
(22) Date of filing: 20.09.2016
(51) Int. Cl.: C12N 15/80

(54) **METHOD FOR SELECTIVE CARBON SOURCE-INDEPENDENT EXPRESSION OF PROTEIN-ENCODING SEQUENCES IN A FILAMENTOUS FUNGUS CELL**
VERFAHREN ZUM AUSWÄHLEN VON SELEKTIVER KOHLENSTOFFQUELLENUNABHÄNGIGER EXPRESSION VON PROTEINCODIERENDEN SEQUENZEN IN EINER FILAMENTÖSEN PILZ-ZELLE
PROCÉDÉ DE SÉQUENCES DE CODAGE DE SOURCE SÉLECTIVE DE CARBONE INDÉPENDANTE DE L'EXPRESSION DE PROTÉINES, DANS UNE CELLULE DE CHAMPIGNON FILAMENTEUSE

(43) Date of publication of application: 21.03.2018
(73) Proprietor: Clariant International Ltd, 4132 Muttenz (CH)
(72) Inventor: GAMAUF, Christian, 81241 München (DE); SEIBOTH, Bernhard, 2102 Bisamberg (AT); KUBICEK, Christian P., 1100 Wien (AT); BISCHOF, Robert, 1080 Wien (AT); SCHIRRMACHER, Georg, 85521 Ottobrunn (DE)
(74) Representative: Graser, Konstanze

(56) References cited:
- WO-A1-2012/083081
- WO-A2-2012/075151
- CN-A- 102 876 706
- CN-A- 104 975 039
- CN-A- 105 602 919
- CN-B- 102 311 951
- CN-B- 103 114 089
- SHOJI J Y ET AL: "Development of Aspergillus oryzae thiA promoter as a tool for molecular biological studies", FEMS MICROBIOLOGY LETTERS, WILEY-BLACKWELL PUBLISHING LTD, GB, vol. 244, no. 1, 1 March 2005 (2005-03-01) , pages 41-46, XP027871657, ISSN: 0378-1097 [retrieved on 2005-03-01]
- DIEGO MARTINEZ ET AL: "Genome sequencing and analysis of the biomass-degrading fungus Trichoderma reesei (syn. Hypocrea jecorina)", NATURE BIOTECHNOLOGY, GALE GROUP INC, US, vol. 26, no. 5, 1 May 2008 (2008-05-01), pages 553-560, XP002689190, ISSN: 1087-0156, DOI: 10.1038/NBT1403 [retrieved on 2008-05-04]
- IRINA S. DRUZHININA ET AL: "A complete annotation of the chromosomes of the cellulase producer Trichoderma reesei provides insights in gene clusters, their expression and reveals genes required for fitness", BIOTECHNOLOGY FOR BIOFUELS, vol. 9, no. 1, 29 March 2016 (2016-03-29), XP055348212, DOI: 10.1186/s13068-016-0488-z
- MACH R L ET AL: "Regulation of gene expression in industrial fungi: Trichoderma", APPLIED MICROBIOLOGY AND BIOTECHNOLOGY, SPRINGER, DE, vol. 60, no. 5, 1 January 2003 (2003-01-01), pages 515-522, XP002506248, ISSN: 0175-7598, DOI: 10.1007/S00253-002-1162-X [retrieved on 2002-12-03]
- GEN ZOU ET AL: "Construction of a cellulase hyper-expression system in Trichoderma reesei by promoter and enzyme engineering", MICROBIAL CELL FACTORIES, BIOMED CENTRAL, GB, vol. 11, no. 1, 8 February 2012 (2012-02-08), page 21, XP021125147, ISSN: 1475-2859, DOI: 10.1186/1475-2859-11-21
- JUNXIN LI ET AL: "Achieving efficient protein expression in Trichoderma reesei by using strong constitutive promoters", MICROBIAL CELL FACTORIES, BIOMED CENTRAL, GB, vol. 11, no. 1, 18 June 2012 (2012-06-18), page 84, XP021115901, ISSN: 1475-2859, DOI: 10.1186/1475-2859-11-84

## Description

The invention relates to a method for selective carbon source-independent expression of protein-encoding sequences in the filamentous fungus cell as well as to the use of such a promotor for selective carbon source-independent expression of protein-encoding sequences in a filamentous fungus cell.

Filamentous fungi are widely used in agro-biochemical processes such as the hydrolysis of cellulosic or lignocellulosic material which is, for example, used as the biomass substrate in bioethanol producing processes. In addition, such hydrolysates can also function as the substrate for the production of many organic compounds such as lactic acid and efficient and controllable hydrolysis is therefore a long-formed need within the state of the art. As hydrolysis is strongly dependent on the production of the hydrolytic enzymes of the filamentous fungus and the respective yields or concentration of the produced enzymes it is further a long-felt need to initiate or to stop the expression of a certain enzyme at a desired time.

It is further necessary to use strong promotors, which make it possible to obtain a sufficient level of the protein of interest at a certain time. Within the art constitutive promotors are used which are e.g. described by Li et al. (Achieving efficient protein expression in Trichoderma reesei by using strong constitutive promoters; Microbial Cell Factories, 2012,11:84). It is, however, preferable, to use so-called inducible promotors which make it possible to initiate or stop the transcription of the gene of interest at a certain time, which is usually linked to a certain process step within e.g. the complex hydrolysis process of lignocellulosic material. As promotors originating from the filamentous fungi are usually not substrate-independent and therefore control of the transcription is difficult, there is currently only a relatively restricted choice of inducible promotors which can be used, in practice, for controlled transcription of genes of interest in filamentous fungi. If the promotors used are not substrate-independent, compounds present in the substrate or produced by other enzymes within the hydrolysis process will influence the transcription of enzymes making control impossible.

Further, such promotors do not only require to be controllable, but also to show a sufficient expression efficiency and also good expression rates. Ideally, the expression should reach a high level under induction conditions and should be able to be completely blocked under non-induction conditions.

In the following is described that these needs can be addressed by a method for selective carbon source-independent expression of protein-encoding sequences in a filamentous fungus cell comprising the steps
a) Introducing a promotor of 1550 basepairs (bp) length comprising the sequence pattern X₁X₂X₃X₄tX₅X₆tcX₇X₈ wherein X₁ is g, t or c, X₂ is a, g, t or c, X₃ is c or t, X₄ is c or t, X₅ is g, a, c or t, X₆ is c or t, X₇ is c or a, X₈ is c or t into the filamentous fungus cell.
b) Contacting the filamentous fungus cell containing the promotor with a growth medium;
c) Adding at least one organic acid to the growth medium.

In particular, the inventors of the present invention have now surprisingly found that these needs can be addressed by a method for selective initiation of expression of a protein-encoding sequence, which is independent of the carbon source used for growth of a filamentous fungus, in a filamentous fungus cell comprising the steps
a) Introducing a promotor of SEQ ID NO:3 into the filamentous fungus cell;
b) Contacting the filamentous fungus cell containing the promotor with a growth medium;
c) Adding at least one organic acid to the growth medium to initiate transcription of the protein-encoding sequence.

The term "carbon source-independent expression" is to be understood as any expression of a protein-encoding sequence which is initiated by a compound or substance not contained within the carbon source used for growth of the filamentous fungus (growth medium) or contained within the carbon source used for growth of the filamentous fungus (growth medium) but in a concentration of at least two orders of the magnitude lower than the carbon-source.

The term "expression" is to be understood as the process by which information from a gene (within the present application "the protein encoding sequence") is used in the synthesis of a functional gene product i.e. the proteins. The process of gene expression is used by all known life-eukaryotes (including multicellular organisms), prokaryotes (bacteria and archaea), and utilized by viruses-to generate the macromolecular machinery for life.

Within the scope of the present invention, the term "carbon source-independent expression" is particularly used for any expression of a protein which is not initiated by or independent from compounds of the respective substrate used for growth of the filamentous fungus cell, but also not initiated by or independent from any compound occurring during the growth process e.g. intermediate products of the hydrolysis process. Therefore, as defined within step b) of the inventive method, at least one organic acid is added to the growth medium in order to initiate transcription of the protein-encoding sequence.

Within a particularly preferred embodiment, the organic acid is selected from the group consisting of ascorbic acid, folic acid, fumaric acid, malic acid, malonic acid, pantothenic acid, phthalic acid, quinic acid, succinic acid, terephthalic acid or derivates thereof and their mixtures. Within the present invention pantothenic acid is particularly preferred.

The term "protein-encoding sequence" is to be understood as pertaining to any protein-encoding sequence known to a person skilled in the art. Within a particularly preferred embodiment the protein is a heterologous protein. Within a further preferred embodiment the protein is a heterologous protein from a bacterial or yeast microorganism. Within a further particularly preferred embodiment the protein-encoding sequence is a sequence encoding for an enzyme of transcription factor. In case the protein-encoding sequence is an enzyme-encoding sequence, the sequence is preferably selected from esterases, hydrolases, lyases, oxidases, recombinases and transferases.

Within the present invention the term "filamentous fungus cell" is to be understood as any cell from any filamentous fungus existing in nature and/or known to a person skilled in the art. The term also comprises any filamentous fungus cell either of natural origin or wild type or genetically modified. Within a preferred embodiment the filamentous fungus cell is selected from the group consisting of *Acremonium, Aspergillus, Chaetomium, Fusarium, Humicola, Irpex, Magnaporte, Myceliophthora, Neurospora, Penicillium, Rhizopus, Talaromyces, Trichoderma* and *Trametes,* wherein *Trichoderma* is particularly preferred.

The promotor described is for example of 1550 base pairs (bp) length and comprises a sequence pattern of X₁X₂X₃X₄tX₅X₆tcX₇X₈ wherein X₁ is g, t or c, X₂ is a, g, t or c, X₃ is c or t, X₄ is c or t, X₅ is g, a, c or t, X₆ is c or t, X₇ is c or a, X₈ is c or t. Within a preferred embodiment the following positions are preferably selected as follows: X₁ is c, X₂ is c, X₃ is t, X₄ is c, X₅ is a or c, X₆ is t or c, X₇ is a or c, X₈ is c. Within a particularly preferred embodiment X₅ is c, X₆ is x, and X₇ is c.

Within another preferred embodiment the promotor comprises one or more of the following bp

| bp upstream of the A in the start codon | base |
|---|---|
| 1 | C |
| 4 | C |
| 204 | A |
| 226 | G |
| 285 | C |
| 297 | G |
| 433 | T |
| 478 | C |
| 650 | C |
| 662 | C |
| 738 | T |
| 851 | A |
| 897 | G |
| 899 | C |
| 1010 | T |
| 1013 | C |
| 1110 | G |
| 1151 | G |
| 1185 | C |
| 1261 | C |
| 1262 | T |
| 1265 | T |
| 1287 | T |
| 1292 | A |
| 1488 | G |
| 1489 | A |

Within an even further preferred embodiment the promotor comprises one or more of the following bp:

| bp upstream of the A in the start codon | base |
|---|---|
| 1 | C |
| 3 | A |
| 4 | C |
| 6 | T |
| 8 | T |
| 12 | A |
| 14 | C |
| 15 | C |
| 16 | T |
| 33 | A |
| 34 | C |
| 35 | T |
| 36 | A |
| 37 | C |
| 39 | A |
| 40 | C |
| 42 | A |
| 45 | C |
| 53 | T |
| 54 | C |
| 56 | C |
| 58 | A |
| 61 | T |
| 62 | T |
| 64 | T |
| 68 | C |
| 69 | A |
| 71 | C |
| 73 | T |
| 75 | C |
| 77 | G |
| 81 | A |
| 82 | G |
| 85 | A |
| 87 | A |
| 88 | T |
| 90 | A |
| 91 | G |
| 92 | A |
| 94 | A |
| 95 | A |
| 96 | C |
| 170 | C |
| 172 | C |
| 173 | C |
| 175 | C |
| 177 | A |
| 178 | C |
| 179 | T |
| 180 | A |
| 181 | C |
| 184 | C |
| 190 | G |
| 195 | C |
| 197 | T |
| 198 | T |
| 204 | A |
| 205 | C |
| 207 | G |
| 209 | C |
| 210 | A |
| 212 | G |
| 213 | T |
| 214 | T |
| 216 | G |
| 217 | T |
| 218 | C |
| 219 | A |
| 220 | C |
| 221 | G |
| 223 | C |
| 226 | G |
| 285 | C |
| 289 | A |
| 290 | G |
| 293 | A |
| 296 | G |
| 297 | G |
| 298 | T |
| 299 | C |
| 300 | G |
| 301 | A |
| 303 | G |
| 383 | A |
| 384 | T |
| 386 | G |
| 389 | C |
| 391 | A |
| 392 | G |
| 393 | T |
| 394 | C |
| 395 | C |
| 399 | G |
| 401 | A |
| 402 | A |
| 421 | G |
| 422 | A |
| 423 | G |
| 424 | G |
| 425 | C |
| 428 | A |
| 431 | C |
| 433 | T |
| 435 | G |
| 436 | G |
| 437 | C |
| 454 | C |
| 455 | G |
| 456 | A |
| 460 | C |
| 461 | T |
| 462 | T |
| 463 | C |
| 472 | A |
| 473 | A |
| 476 | G |
| 478 | C |
| 479 | T |
| 481 | C |
| 482 | G |
| 483 | A |
| 484 | A |
| 489 | G |
| 490 | A |
| 491 | G |
| 493 | G |
| 495 | G |
| 497 | C |
| 498 | T |
| 499 | C |
| 502 | T |
| 504 | C |
| 505 | A |
| 506 | G |
| 507 | C |
| 589 | G |
| 590 | C |
| 591 | A |
| 594 | A |
| 595 | A |
| 597 | G |
| 598 | A |
| 600 | C |
| 601 | A |
| 602 | C |
| 603 | T |
| 604 | A |
| 605 | C |
| 606 | T |
| 607 | G |
| 628 | A |
| 629 | G |
| 632 | T |
| 633 | A |
| 634 | A |
| 635 | A |
| 636 | A |
| 637 | G |
| 639 | A |
| 649 | C |
| 650 | C |
| 651 | T |
| 652 | T |
| 655 | A |
| 658 | G |
| 662 | C |
| 663 | C |
| 668 | G |
| 669 | T |
| 670 | A |
| 674 | G |
| 675 | G |
| 676 | G |
| 677 | A |
| 682 | G |
| 684 | G |
| 685 | A |
| 686 | G |
| 687 | G |
| 688 | A |
| 693 | T |
| 694 | A |
| 696 | A |
| 697 | G |
| 736 | G |
| 737 | C |
| 738 | T |
| 739 | A |
| 742 | A |
| 743 | G |
| 744 | A |
| 745 | T |
| 746 | G |
| 747 | G |
| 748 | T |
| 749 | G |
| 750 | T |
| 752 | G |
| 753 | A |
| 754 | A |
| 755 | T |
| 757 | G |
| 759 | A |
| 776 | T |
| 780 | G |
| 782 | T |
| 784 | A |
| 785 | T |
| 786 | C |
| 787 | G |
| 804 | T |
| 805 | A |
| 806 | G |
| 810 | G |
| 811 | G |
| 813 | G |
| 816 | T |
| 817 | T |
| 819 | G |
| 821 | G |
| 822 | G |
| 845 | G |
| 847 | G |
| 848 | C |
| 850 | G |
| 851 | A |
| 853 | T |
| 854 | A |
| 856 | G |
| 857 | G |
| 860 | A |
| 869 | G |
| 870 | T |
| 871 | T |
| 872 | A |
| 874 | A |
| 875 | A |
| 879 | G |
| 880 | T |
| 886 | C |
| 888 | G |
| 889 | C |
| 890 | G |
| 892 | G |
| 897 | G |
| 898 | T |
| 899 | C |
| 900 | T |
| 920 | A |
| 922 | G |
| 923 | G |
| 925 | G |
| 926 | G |
| 928 | G |
| 929 | T |
| 931 | C |
| 932 | C |
| 941 | G |
| 942 | G |
| 943 | C |
| 945 | A |
| 946 | G |
| 948 | G |
| 969 | G |
| 970 | A |
| 971 | A |
| 972 | C |
| 1002 | G |
| 1004 | C |
| 1005 | T |
| 1006 | T |
| 1009 | T |
| 1010 | T |
| 1013 | C |
| 1016 | G |
| 1017 | G |
| 1064 | G |
| 1065 | G |
| 1066 | C |
| 1067 | C |
| 1068 | G |
| 1069 | T |
| 1071 | A |
| 1073 | T |
| 1077 | C |
| 1082 | A |
| 1084 | A |
| 1085 | T |
| 1087 | A |
| 1089 | C |
| 1091 | G |
| 1092 | C |
| 1096 | G |
| 1102 | A |
| 1104 | C |
| 1106 | A |
| 1108 | C |
| 1110 | G |
| 1112 | C |
| 1148 | T |
| 1149 | A |
| 1150 | C |
| 1151 | G |
| 1152 | G |
| 1153 | C |
| 1154 | G |
| 1156 | G |
| 1157 | A |
| 1158 | C |
| 1159 | A |
| 1161 | A |
| 1164 | T |
| 1168 | A |
| 1169 | C |
| 1170 | C |
| 1172 | T |
| 1173 | A |
| 1174 | C |
| 1177 | A |
| 1182 | G |
| 1183 | A |
| 1185 | C |
| 1186 | A |
| 1188 | C |
| 1189 | T |
| 1191 | C |
| 1196 | G |
| 1198 | T |
| 1199 | A |
| 1200 | A |
| 1245 | G |
| 1246 | C |
| 1249 | C |
| 1250 | A |
| 1251 | C |
| 1252 | A |
| 1253 | C |
| 1256 | C |
| 1259 | C |
| 1261 | C |
| 1262 | T |
| 1265 | T |
| 1266 | C |
| 1267 | T |
| 1268 | C |
| 1269 | C |
| 1271 | C |
| 1274 | C |
| 1277 | A |
| 1284 | C |
| 1285 | A |
| 1286 | G |
| 1287 | T |
| 1292 | A |
| 1297 | A |
| 1298 | G |
| 1299 | T |
| 1302 | G |
| 1303 | T |
| 1305 | T |
| 1320 | G |
| 1323 | A |
| 1324 | A |
| 1325 | G |
| 1326 | A |
| 1329 | G |
| 1330 | T |
| 1334 | T |
| 1336 | C |
| 1337 | C |
| 1340 | G |
| 1341 | A |
| 1346 | T |
| 1348 | G |
| 1355 | C |
| 1356 | T |
| 1357 | A |
| 1358 | C |
| 1359 | A |
| 1360 | T |
| 1361 | C |
| 1362 | G |
| 1363 | T |
| 1365 | G |
| 1368 | C |
| 1375 | A |
| 1378 | T |
| 1381 | T |
| 1384 | A |
| 1385 | G |
| 1386 | G |
| 1387 | T |
| 1421 | T |
| 1422 | G |
| 1423 | A |
| 1425 | T |
| 1428 | G |
| 1429 | A |
| 1470 | A |
| 1471 | C |
| 1472 | T |
| 1473 | A |
| 1476 | G |
| 1477 | A |
| 1480 | G |
| 1482 | T |
| 1483 | G |
| 1486 | G |
| 1488 | G |
| 1489 | A |
| 1544 | G |
| 1545 | G |
| 1546 | A |
| 1547 | A |
| 1549 | T |
| 1550 | T |

Such promotor is selected from the group consisting of SEQ ID NO:1, SEQ ID NO:2, SEQ ID NO: 4 and SEQ ID NO: 5. The promotor of the present invention is a promotor of SEQ ID NO:3-

After the introduction of the respective promotor into the filamentous fungus cell, the filamentous fungus cell is contacted with a growth medium according to step b) of the invented method. The "contacting" can be carried out to any method known to a person skilled in the art as suitable for the inventive purpose. Within a preferred embodiment the filamentous fungus cell is added to a growth medium. The growth medium is preferably comprising cellulose, lignocellulose and/or glucose as carbon source. An example for a preferred growth medium is Mandels-Andreotti medium.

According to step c) of the method of the present invention at least one organic acid is added to the growth medium. The "adding" according to step c) can be carried out by any method known to a person skilled in the art as suitable for the inventive purpose. The at least one organic acid is preferably added in an amount of from 0.1 to 500 mM, preferably of from 1 to 75 mM and particularly preferred of from 5 to 50 mM. In case *Trichoderma reesei* is chosen as the filamentous fungus cell the at least one organic acid is added in an amount of from 1 to 50 mM, preferably 5 to 15 mM in case pantothenic acid is used and in case quinic acid is used, the quinic acid is added in an amount of from 10 to 500 mM, preferably 50 to 150 mM.

Within a further preferred embodiment the promotor is introduced into the filamentous fungus cell within a vector. Within the present invention the term "vector" is used to define any vehicle functioning to stably deliver the promotor sequence fused to a reporter gene into the filamentous fungus cell. Preferred vectors have the ability to replicate by themselves and to be transformed into the filamentous fungal cell and preferably also include a detectable portion. Vectors preferred are for example plasmid which may also be delinearized or digested prior to transformation.

Introduction of the promotor into the filamentous fungus cell is carried out according to any method known to a person skilled in the art as suitable for the inventive purpose. Such a method is for exampled described in Penttilä et al. (1987) Gene 61: 155-164 or Gruber et al. (1990) Curr Genet 18: 71-76.

In a further aspect, the present invention is also directed to the use of a promotor as defined before for selective, carbon source-independent expression of protein-encoding sequences in a filamentous fungus cell.

### Figures and examples

The present invention is now described by the following figures and examples. It is thereby emphasized that the figures and examples do not limit the scope of the invention and claims but merely constitute further illustration of the invention, inventive purpose and benefits achieved by the inventive method.

### Figure legends:

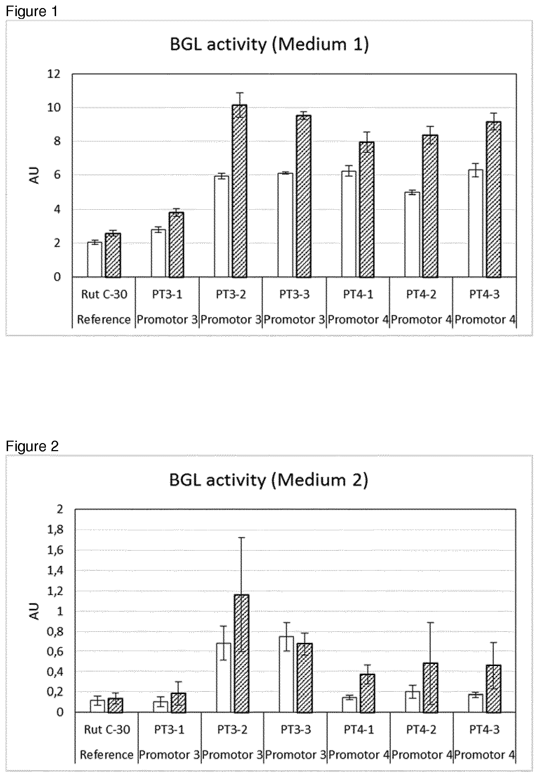

- Figure 1:: Shows the results of the beta glucosidase (BGL) assay of the supernatants in medium 1. The white bars indicate the activity without addition of pantothenic acid as inducer, the shaded bars indicate the activity with pantothenic acid added to the respective cultures. y-Axis: Activity units (AU) calculated as describend in example 1.
- Figure 2:: Shows the results of the beta glucosidase (BGL) assay of the supernatants in medium 2. The white bars indicate the activity without addition of pantothenic acid as inducer, the shaded bars indicate the activity with pantothenic acid added to the respective cultures. y-Axis: Activity units (AU) calculated as describend in example 1.

### Example 1:

### General

The examples show the performance of 4 different promotors including claimed promotor of SEQ ID NO:3 in *Trichoderma reesei* strains cultivated on two different media with beta glucosidase (SEQ ID NO: 6) as protein encoding sequence in comparison to the performance of the respective *Trichoderma reesei* strain without the promotor.

### Construction of the expression vectors

Standard methods known to those skilled in the art and described e.g. by Sambrook and Russel (Molecular Cloning - A laboratory manual; Cold Spring Harbor Laboratory Press, New York) or by Jansohn et al. (Gentechnische Methoden, Elsevier, München) were used for DNA agarose gel electrophorese, purification of DNA, transformation of *Escherichia coli,* plasmid propagation and purification, amplification of pieces of DNA by polymerase chain reaction (PCR) and isolation of genomic DNA from *Trichoderma reesei.* Ligation-independent cloning (LIC) was done essentially as described by Aslanidis and de Jong (1990, Nucleic Acid Res. 18 (20), 6069).

Promotor 1 (SEQ ID NO: 1) was amplified by PCR using genomic DNA from *Trichoderma reesei* as template, primers 1fw (5'-ACTCCATCACTACTGCTTACTATTCTCAAAGGGCCGTTTAC -3') and 1rv (5'-CCAACTTCCTATACATGTTCGAGGCGTCTCCGCTAATG -3') and phusion polymerase from Thermo Fisher Scientific according to the manufacturer's instructions (annealing temperature: 72 °C, elongation time: 45 sec). The amplicon was purified using the Wizard PCR purification kit from Promega.

Promotor 2 (SEQ ID NO:2) was amplified by PCR using genomic DNA from *Trichoderma reesei* as template, primers 2fw (5'- ACTCCATCACTACTGATTGCAGACACTTGGACTTG - 3') and 2rv (5'- CCAACTTCCTATACATGCTTGAAGGAGTGAAGTAGATAGG -3') and phusion polymerase from Thermo Fisher Scientific according to the manufacturer's instructions (annealing temperature: 60.9 °C, elongation time: 25 sec). The amplicon was purified using the Wizard PCR purification kit from Promega.

Promotor 3 (SEQ ID NO: 3) of the present invention was amplified by PCR using genomic DNA from *Trichoderma reesei* as template, primers 3fw (5'-ACTCCATCACTACTGCAGAACCGTGCCATTTTC -3') and 3rv (5'-CCAACTTCCTATACATGGTGCATATCATGGGATGAG -3') and phusion polymerase from Thermo Fisher Scientific according to the manufacturer's instructions (annealing temperature: 65.9 °C, elongation time: 50 sec). The amplicon was purified using the Wizard PCR purification kit from Promega.

Promotor 4 (SEQ ID NO:4) was amplified by PCR using genomic DNA from *Trichoderma reesei* as template, primers 4fw (5'-ACTCCATCACTACTGCTTGAAGGAGTGAAGTAGATAGG -3') and 4rv (5'-CCAACTTCCTATACATGATTGCAGACACTTGGACTTG -3') and phusion polymerase from Thermo Fisher Scientific according to the manufacturer's instructions (annealing temperature: 60.9 °C, elongation time: 25 sec). The amplicon was purified using the Wizard PCR purification kit from Promega.

Promotor 5 (SEQ ID NO:5) was amplified by PCR using genomic DNA from *Trichoderma reesei* as template, primers 5fw (5'- ACTCCATCACTACTGCCAAATCGGGCAGAG -3') and 5rv (5'- CCAACTTCCTATACATGCTTGTGCTGCCAAGGTGAGAG -3') and phusion polymerase from Thermo Fisher Scientific according to the manufacturer's instructions (annealing temperature: 67.4 °C, elongation time: 45 sec). The amplicon was purified using the Wizard PCR purification kit from Promega.

Plasmid pPromotorTest (SEQ ID NO:7) was digested with Acul (from New England Biolabs) according to the manufacturer's instructions and purified using the Wizard PCR purification kit from Promega.

Promotors 1 to 5 (amplified by PCR as described above) were fused with linearized pPromotorTest using ligation independent cloning (LIC). The linearized vector was treated with T4 DNA polymerase in the presence of dGTP. The amplified promotors were treated with T4 DNA polymerase in the presence of dCTP. T4 DNA polymerase treated vector and promotors were mixed and annealed as described in Ref. The assays were then transformed in chemically competent *Escherichia coli* XL1-Blue cells, plated on LB-Agar plates containing 100 mg·l⁻¹ ampicillin (LB-Amp) and incubated at 37 °C for 24 h. Colonies were picked from the agar plates using toothpicks, transferred into liquid LB-Amp medium and incubated at 37 °C for 24 h with shaking (250 RPM). Plasmid DNA was isolated and integration of the insert was verified by PCR using the primers Testfw (5'- GAGCAATGTGGGACTTTGATG -3') and Testrv (5'- GCCCAATCTTGGGATGCTAC -3') and the GoTaq Green master mix from Promega according to the manufacturer's instructions (annealing temperature: 60 °C, elongation time: 2 min 45 sec). Plasmids that gave the expected band (Promotor 1: 2.5 kb, Promotor 2: 1.8 kb, Promotor 3: 2.7 kb, Promotor 4: 1.8 kb, Promotor 5: 2.6 kb, empty vector: 1.1 kb) were verified by sequencing and named pPTest-1 to -5 accordingly.

### Transformation of the promotor test vectors into Trichoderma reesei

Vectors pPTest-1 to -5 were digested with Sbfl (from New England Biolabs) according to the manufacturer's instructions. *Trichoderma reesei* Rut C-30 (CBS 820.91) was transformed with the digested vectors essentially as described in Penttilä et al (1987) Gene 61: 155-164 or Gruber et al (1990) Curr Genet 18: 71-76. The transformants were selected on potato dextrose agar plates containing 250 mg·l⁻¹ of hygromycin and 1 M sorbitol and purified by singularisation. Conidia stocks of the purified strains were prepared by growing them on potato dextrose agar plates at 30 °C until the plates were covered with spores. The conidia were harvested with sterile sodium chloride (0.9 g·l⁻¹)-Triton X-100 (0.01 mg·l⁻¹) solution, adjusted to OD₆₀₀ = 10, supplemented with 50 g·l⁻¹ of glycerol and stored at -80 °C.

Genomic DNA was isolated from the mycelium of the strains. The recombinant promotor regions were amplified by PCR using the genomic DNAs as templates, primer intfw (5'-GCACAACCGCATGATATAGGG-3') and intrv (5'- CCCAATCTTGGGATGCTACC-3') and phusion polymerase from Thermo Fisher Scientific according to the manufacturer's instructions (annealing temperature: 65.7 °C, elongation time: 1 min 35 sec). The amplicons were purified using the Wizard PCR purification kit from Promega and sequenced.

Strains containing the vector pPTest-1 were named PT1-1 to -3.

Strains containing the vector pPTest-2 were named PT2-1 to -3.

Strains containing the vector pPTest-3 were named PT3-1 to -3.

Strains containing the vector pPTest-4 were named PT4-1 to -3.

Strains containing the vector pPTest-5 were named PT5-1 to -3.

### Growth of the reporter strains in shake flasks

The strains were grown in shake flasks in two media:
Medium 1 contains (mg·l⁻¹): (NH4)2SO4 1.4, KH2PO4 2.0, MgSO4 * 7 H20 0.3, CaCl₂ * 2 H2O 0.3, potassium phthalate 10.2, FeSO4 * 7 H2O 0.005, MnSO4 * H2O 0.0016, ZnSO4 * 7 H2O 0.0014, CuSO4 * 5 H2O 0.0001, Avicel (= microcrystalline cellulose) 20 and milled residue from extracted soy beans 5. The medium was adjusted to pH 5 with HCI or NaOH.
Medium 2 contains (mg·l⁻¹): (NH4)2SO4 2.8, KH2PO4 2.0, MgSO4 * 7 H20 0.3, CaCl2 * 2 H2O 0.3, potassium phthalate 10.2, FeSO4 * 7 H2O 0.02, MnSO4 * H2O 0.0064, ZnSO4 * 7 H2O 0.0056, CuSO4 * 5 H2O 0.0004, glucose 10 and yeast extract 0.5. The medium was adjusted to pH 5.5 with HCl or NaOH.

15 ml of the media were distributed into 50 ml Erlenmeyer shake flasks under a sterile hood and the flasks were closed with rubber foam caps. Conidia stocks of the transformants and of strain Rut C-30 were thawed and 75 µl of the conidia suspensions were pipetted into the erlenmeyer flasks with the media under a sterile hood. Six flasks were inoculated per strain and medium. The flasks were incubated at 30 °C with shaking (250 RPM) for 6 (Medium 1) or 3 (Medium 2) days. To test the induction of the promotors 10 mM pantothenic acid were added to three flasks per strain and medium after 48 (Medium 1) or 24 h (Medium 2).

The cultures were poured into 15 ml tubes, centrifuged (3220xg, 4 °C, 15 min), the supernatants transferred into new tubes and stored at 4 °C.

### Analysis of beta-alucosidase activity in the culture supernatants

Since a beta-glucosidase (BGL) is used as a reporter gene to test the promotor activity, the BGL activity in the culture supernatants was measured to determine the activity of the promotors integrated in the respective strains. To measure the activity, the supernatants were first diluted in acetate buffer (50 mM; pH 5.0), if necessary. 50 µl of the samples were mixed with 50 µl of 2 mM p-nitrophenol-beta-D-glucopyranoside (dissolved in acetate buffer) and incubated at 60 °C for 30 min. Then 100 µl of 1 M Na2CO3 (dissolved in water) were added, and the absorption at 405 nm was measured using a spectrophotometer. Activity units (AU) were calculated by multiplying the absorbance values (A405) and the dilution.

The results are shown in Figures 1 and 2.

It can be seen from the figures and examples that the promotor of SEQ ID NO:3 which comprises the inventive sequence pattern fulfills the claimed benefits and leads to a significant improved protein-of-interest expression under induction conditions.

## Claims

1. Method for selective initiation of expression of a protein-encoding sequence, which is independent of the carbon source used for growth of a filamentous fungus, in a filamentous fungus cell comprising the steps
a) Introducing a promotor of SEQ ID NO:3 into the filamentous fungus cell;
b) Contacting the filamentous fungus cell containing the promotor with a growth medium;
c) Adding at least one organic acid to the growth medium to initiate transcription of the protein-encoding sequence.

2. Method according to claim 1, wherein the protein is a heterologous protein.

3. Method according to claim 1 or 2, wherein the filamentous fungus is selected from the group of *Acremonium, Aspergillus, Chaetomium, Fusarium, Humicola, Irpex, Magnaporte, Myceliophthora, Neurospora, Penicillium, Rhizopus, Talaromyces, Trichoderma and Trametes.*

4. Method according to any of the foregoing claims, wherein the organic acid is selected from ascorbic acid, folic acid, fumaric acid, malic acid, malonic acid, pantothenic acid, phthalic acid, quinic acid, succinic acid, terephthalic acid or derivates thereof.

5. Method according to any of the foregoing claims, wherein protein-encoding sequence is a sequence encoding for an enzyme or transcription factor.

6. Method according to any of the foregoing claims wherein the enzyme-encoding sequence is selected from the group consisting of esterases, hydrolases, lyases, oxidases, recombinases, transferases.

7. Method according to any of the foregoing claims wherein the promotor is introduced within a vector.

8. Use of a promotor as defined in any of the foregoing claims for selective, carbon source-independent expression of protein-encoding sequences in a filamentous fungus cell.

## Patentansprüche

1. Verfahren zur selektiven Initiation der Expression einer Protein codierenden Sequenz, die unabhängig von der zur Kultur eines Fadenpilzes verwendeten Kohlenstoffquelle ist, in einer Fadenpilzzelle, umfassend die Schritte
a) Einführen eines Promotors unter SEQ ID NO:3 in die Fadenpilzzelle;
b) Inkontaktbringen der den Promotor enthaltenden Fadenpilzzelle mit einem Kulturmedium;
c) Zugeben wenigstens einer organischen Säure zum Kulturmedium zur Initiation der Transkription der Protein codierenden Sequenz.

2. Verfahren nach Anspruch 1, wobei es sich bei dem Protein um ein heterologes Protein handelt.

3. Verfahren nach Anspruch 1 oder 2, wobei der Fadenpilz ausgewählt ist aus der Gruppe bestehend aus *Acremonium, Aspergillus, Chaetomium, Fusarium, Humicola, Irpex, Magnaporte, Myceliophthora, Neurospora, Penicillium, Rhizopus, Talaromyces, Trichoderma* und *Trametes.*

4. Verfahren nach einem der vorhergehenden Ansprüche, wobei die organische Säure ausgewählt ist aus Ascorbinsäure, Folsäure, Fumarsäure, Äpfelsäure, Malonsäure, Pantothensäure, Phthalsäure, Chinasäure, Bernsteinsäure, Terephthalsäure oder Derivaten davon.

5. Verfahren nach einem der vorhergehenden Ansprüche, wobei es sich bei der Protein codierenden Sequenz um eine Sequenz handelt, die für ein Enzym oder einen Transkriptionsfaktor codiert.

6. Verfahren nach einem der vorhergehenden Ansprüche, wobei die Enzym codierende Sequenz ausgewählt ist aus der Gruppe bestehend aus Esterasen, Hydrolasen, Lyasen, Oxidasen, Rekombinasen, Transferasen.

7. Verfahren nach einem der vorhergehenden Ansprüche, wobei der Promotor in einem Vektor eingeführt wird.

8. Verwendung eines Promotors gemäß einem der vorhergehenden Ansprüche zur selektiven, karbonsäureunabhängigen Expression Protein codierender Sequenzen in einer Fadenpilzzelle.

## Revendications

1. Procédé pour l'initiation sélective de l'expression d'une séquence codant pour une protéine, qui est indépendante de la source de carbone utilisée pour la croissance d'un champignon filamenteux, dans une cellule de champignon filamenteux comprenant les étapes
a) introduction d'un promoteur de SEQ ID n° : 3 dans la cellule de champignon filamenteux ;
b) mise en contact de la cellule de champignon filamenteux contenant le promoteur avec un milieu de croissance ;
c) ajout d'au moins un acide organique au milieu de croissance pour initier la transcription de la séquence codant pour une protéine.

2. Procédé selon la revendication 1, dans lequel la protéine est une protéine hétérologue.

3. Procédé selon la revendication 1 ou 2, dans lequel le champignon filamenteux est choisi dans le groupe de *Acremonium, Aspergillus, Chaetomium, Fusarium, Humicola, Irpex, Magnaporte, Myceliophthora, Neurospora, Pénicillium, Rhizopus, Talaromyces, Trichoderma* et *Trametes.*

4. Procédé selon l'une quelconque des revendications précédentes, dans lequel l'acide organique est choisi parmi l'acide ascorbique, l'acide folique, l'acide fumarique, l'acide malique, l'acide malonique, l'acide pantothénique, l'acide phtalique, l'acide quinique, l'acide succinique, l'acide téréphtalique ou les dérivés de ceux-ci.

5. Procédé selon l'une quelconque des revendications précédentes, dans lequel la séquence codant pour une protéine est une séquence codant pour une enzyme ou un facteur de transcription.

6. Procédé selon l'une quelconque des revendications précédentes dans lequel la séquence codant pour une enzyme est choisie dans le groupe constitué par les estérases, les hydrolases, les lyases, les oxydases, les recombinases, les transférases.

7. Procédé selon l'une quelconque des revendications précédentes dans lequel le promoteur est introduit au sein d'un vecteur.

8. Utilisation d'un promoteur tel que défini dans l'une quelconque des revendications précédentes pour l'expression sélective indépendante de la source de carbone de séquences codant pour une protéine dans une cellule de champignon filamenteux.


### SEQUENCE LISTING

### SEQUENCE LISTING

<110> Clariant International Ltd
<120> Method for selective carbon source-independent expression of
   protein-encoding sequences in a filamentous fungus cell
<130> R6488
<160> 7
<170> BiSSAP 1.3.6
<210> 1
   <211> 1379
   <212> DNA
   <213> Trichoderma reesei
<400> 1
   cttactattc tcaaagggcc gtttacgggg ctatataagg agcaaatcaa cggaatacag      60
   gctgtgtgtg tagcaggaaa tggatcggcg aacggggggt tcttctcttc tgtcatgatt     120
   tgttgagata gggccagcgg aaactagttt gtcacttcac ttctaattgt gagtccaaaa     180
   acgataatct caatgtgttc ttcatccatg aacaaatcgt acgtaacgtt gtcttgcatg     240
   catacaatca tggccgtata agccaccgag ctgctccttt gatgcaactg tgcgtccggg     300
   ctgccgagat acgcctatcc agacatatcc accccgttgg cgctactatc acagatcagc     360
   tggatcatgg tcaaagtcgc tgaggctaac aggttgtcgc cgccttcgcg tgagcctagc     420
   attcagtttg ctaatgcttc tgccctgcta ctggccttga tcgttatcca ccagaccagt     480
   caaattgcaa gtaaaagcaa gttccaattc gtatcaacgc atcagtcatg tcattcacat     540
   cacctacgca atctggatca ccatctgcat aaactggggt tgttgacctc ggtatcaagc     600
   caccggcgac tagtcgagat aacgcccctc ctcctccacc atctccgcaa ctggataaac     660
   gaccccaaga gacatcggaa cttagaacag ccatcaacca taccgtaaca ggcaatcaat     720
   ctattgtcgt catacagtca gcatctcctc atcactgact gctgttatga tttggggaag     780
   acatcccagc ggctagacga cggaccagat ccgaggcaaa gaacttcttg agacggcccc     840
   catcccaatt cctaacaagg ttgatccacc ttctgcatca caagaattag catcatctca     900
   gtcgtccccc tcttgagctg cccgtatgca atcttcgtct cggcatccgg gacccattgg     960
   caacccggga tatgcgagag aaaccccgca ctgggcctca ctttgaagca tatggatcga    1020
   ttccaaagtc caactggtgt cgaatcgcaa aaacgtaccg ctttcgtcgt atcaaaggac    1080
   gagggatccg gcatcgggtg gcggcaatga tatcaagctg agtcttggcg ttatgaactg    1140
   tttcctttta tatttgtctg aagagagaga gaccacgagg ccaaaggatt tacattacag    1200
   cacaagaaac aggggacgac ctctttgctg gcggttatac tttgtttcat cctcctcaca    1260
   cagcccacgt tttttcgatc ataactgttt cttgccctca tcacaacgcc tcccctattc    1320
   cctacctacc ctctcatcca atacttggtt gatacattag cggagacgcc tcgaacatg 1379
<210> 2
   <211> 728
   <212> DNA
   <213> Trichoderma reesei
<400> 2
   attgcagaca cttggacttg atgctggtaa gagaggtaaa acacaagtag caagactctg 60
   tctattcttt tggtggtggt gatggtgttt tgccctggta agatactggc cgtcttacaa 120
   ccagtgctgc tcgtgttcgc tctccagctg cttaactagc tcagggctcg ttctccgggt 180
   ccgaaaccgg cttcagaaga ttgaccgaag cttcagcctc ccgtcccgag acaagtcgtc 240
   gtggttcctg tgatgactcg tattttcttg gaaattaccc tcgggccatt acccatgaat 300
   cactcctcct gatgtcccga ttctctacca caacttacct tagtactagt agcatctccc 360
   cgcacaaact ccccgcgact tattcccgta gcaggcaata ttgtacagac gccctttaca 420
   gacttccccc acacggccga tctccttggc tgaagaaaaa gagccccggc actaatacgt 480
   ttagttgacg gatctaaaat agctggacag atgccgcact gtatacagtt tggatgtttc 540
   tttcttgttg atgaaaccaa ttcgaggtgt gatggggagg agaggggttg actgtcacac 600
   atgatatcag acaaaagggt ccaattcttg cagctacggc cctcactatc tttgatgtag 660
   caactaggtt atcttcatta cttccaactc acactttgat acctatctac ttcactcctt 720
   caagcatg 728
<210> 3
   <211> 1568
   <212> DNA
   <213> Trichoderma reesei
<400> 3
   cagaaccgtg ccattttcaa ggacgccagc gacgatgact atgtcatcgt aaccaaagag      60
   accatattcc atccgcaggg cggtggccag ccatcagacg agggcacaat ctcaacagcc     120
   ccttcaacag acgcccagac tcagtcgccc attgccatca acatcagggc agcacgcatg     180
   gacgttgtca atgacggcgt ggtgctacat ctcggtcgct ttgaagatcc ctcaatggca     240
   gaaaggtttc aggcgggaga tctggttgaa cagaagattg acgccgagaa gcgcctcctc     300
   tattcacgac tgcacactgc gggccatgtt cttggctcag ccgtgaggca tcttttggaa     360
   aaggaaatcg acggcttcga cgagctcaag gcatcccact ttccagacag cgcggcatgc     420
   gagttccgcg gactgattga gggcaagtgg aagcagccga tccaggaccg cgtcgacgag     480
   tacatccgcc gctcaatgcc ggtagaaatc gacttttgga cagaagacga cttccgacgc     540
   gagggactgg agcgcttgat tccggacaag agcctgttgc cgcctggaga gaccaagttc     600
   cgcgtcgtgc ggattgtggg agcggaggtt tatccctgcg gagggacgca tgttgacacg     660
   acggatctgt gcggcgcgac gacggtgaag aagattggga ggagcaaagg cattagccgg     720
   gtcagttaca cggtggcttg acgggggatt caggggaccg atagcggacg aagataaagc     780
   tattggcgat gtcgtcgtgc cggtggagga taagatgtgg tagatgatcg agtatgcgcg     840
   cattccgatg ctggagttag tagatgtaga tatatacagg agggggttag ggacgatgac     900
   cacccctgac atattccata tgggagatga aaattagaca gatagaccac agcccgctgt     960
   catcaccagc aaatacgtcc aacggtatcc atctttcctg ctcctgtgat gcaggatgtg    1020
   gctttgcgaa gcgcatcagc cgatctaaaa acaatctccg acctcgctca gggtgagaaa    1080
   taagcatccg ataaggtgat ggcttcagca gctcccgttt tccatccgcg gatgcatagg    1140
   cggaggtagt cagcgccatc agtaaggttg cctgatccgg atagctgtca tccaaaacgc    1200
   cacagccgat tcgaaaactt gtgaaacttg aagctattgc caatactctg ctacggccac    1260
   cggaagctgg cgatagattg cctacttctt acggcgtagc tgcgggaagc caccgggaac    1320
   cttgaaagtt ggaggttgtg gcccgcactg tttgaacaga cactgagttc caccagcctc    1380
   tccgcatcat ccccgcatga actgagattc gacgtcaggc ctgtacgagc atagaacagg    1440
   tgaagttaca aagcataatg gtggagatgc aagagactac attgatctga ccttcaaccc    1500
   ttcttgcagc gctaccacgg ccaaacacca tcattctgcg gacacctcat cccatgatat    1560
   gcaccatg                                                              1568
<210> 4
   <211> 728
   <212> DNA
   <213> Trichoderma reesei
<400> 4
   cttgaaggag tgaagtagat aggtatcaaa gtgtgagttg gaagtaatga agataaccta 60
   gttgctacat caaagatagt gagggccgta gctgcaagaa ttggaccctt ttgtctgata 120
   tcatgtgtga cagtcaaccc ctctcctccc catcacacct cgaattggtt tcatcaacaa 180
   gaaagaaaca tccaaactgt atacagtgcg gcatctgtcc agctatttta gatccgtcaa 240
   ctaaacgtat tagtgccggg gctctttttc ttcagccaag gagatcggcc gtgtggggga 300
   agtctgtaaa gggcgtctgt acaatattgc ctgctacggg aataagtcgc ggggagtttg 360
   tgcggggaga tgctactagt actaaggtaa gttgtggtag agaatcggga catcaggagg 420
   agtgattcat gggtaatggc ccgagggtaa tttccaagaa aatacgagtc atcacaggaa 480
   ccacgacgac ttgtctcggg acgggaggct gaagcttcgg tcaatcttct gaagccggtt 540
   tcggacccgg agaacgagcc ctgagctagt taagcagctg gagagcgaac acgagcagca 600
   ctggttgtaa gacggccagt atcttaccag ggcaaaacac catcaccacc accaaaagaa 660
   tagacagagt cttgctactt gtgttttacc tctcttacca gcatcaagtc caagtgtctg 720
   caatcatg 728
<210> 5
   <211> 1466
   <212> DNA
   <213> Trichoderma reesei
<400> 5
   ccaaatcggg cagaggcaaa ggcaagggtg ggagggcgga aagggggaaa gataggtggc 60
   aaagaaagga ataagtgtgg aaaagagaag agaaggagag agatcagggg gtggaatgag 120
   agtttggttt agtttagcag acgttgatgt gtctcctggt gcggataaaa gggcttgtat 180
   tagccaggta tcctatgtat gtaggcgagg gagaaggatt tcgggttggt tcagccgtat 240
   gcggggggtt tctttttctc ctctcgttgc agtgctcgtt atcgcagctc tataggccga 300
   gtacgactct ggttattaat tgcccgggaa gactctagta ggcctagtgc tattacacgg 360
   gaaggcatgt tgggggggaa atactactta acaggaggaa gacatgccga atgtggtcca 420
   tagttgagga agaaagataa ctcaacggac cttgaccttg tgtataagag tctttggccc 480
   cccatagact aggtaggtaa cgaactacct tacctaccta gctaccccag cttttcgtat 540
   tctgttgcct ttccaaagag gggactgttc gccgccttgt gcttccccac gttttgccat 600
   gacctcgccg gcccggtgac tggctgataa acgcccagta agtggcttaa gaccaccagc 660
   agacgcggct gctagtgcta tcgtttccag gcttcaagaa gctaaggtac tgccgtatgc 720
   gctgcccacg aaggccaaag gaaatggctg gatgaatcaa cgctagatag ctagattcca 780
   tgccatacca caagcagata ttgtgaagct ggcgaacttg ttagttccgg aaccattgga 840
   gaccctcgag cctgttctag cagccaagcc tttcccggca gaaagggtgt tatctggcgc 900
   cctccgcgac tccagattcc agctccgtca ccacttcacg caacaggatg tcggaagggc 960
   gcgaagctcc gggcctgaac gaccatgagg aaagttcaac ttggcactga gtagctggaa 1020
   acctggctat cgtactgccc aggttgctaa actagagctt ggagataacg aagagaggcc 1080
   accgggctta tcttgccact aggatattcg cacagtccaa gctttttacc tttgcccttc 1140
   gtgtcattgc cgctattcct ccccgtggaa catcatgggg ttggcaatcg tttccttagc 1200
   ggccgaattt ggggattctt tgctagacgg gcagcgattt tccgacggag gagccggttg 1260
   atgcatcttt gaaggatcat aaagatgcag caaacaccct tctttatttc ccctgaggtg 1320
   attcaccgcc aaggcaaatc ttctgcgagg gtgtgttttt gttccatagc cctcattggc 1380
   gagagtaaga caaagatttt tttcccccat acagccgcgt ctctccaaag ggaaacacga 1440
   cctctcacct tggcagcaca agcatg 1466
<210> 6
   <211> 2613
   <212> DNA
   <213> Artificial Sequence
<220>
   <223> BGL from Talaromyces emersonii with TrCBH1 signal peptide and 6xHis-Tag
<400> 6
   atgtatagga agttggccgt catctcggcc ttcttggcca cagctcgtgc ccagagcggc      60
   gccctagaga acctggcgta ttcacctccc ttctaccctt cgccgtgggc caatggacag     120
   ggcgactggg cagaggccta cgagaaggcc gtcaagtttg tctcccaact cacgctggcc     180
   gaaaaggtca acctgaccac cggaactggt tgggagcagg accgatgcgt cggccaagtg     240
   ggtagcatcc caagattggg cttcccagga ctttgcatgc aggactctcc gctgggtgtt     300
   cgagacactg actacaactc ggccttcccg gcgggtgtca atgtcgctgc tacctggaac     360
   agggacctcg cctaccgtcg cggccaagcg atgggcgagg agcatcgcgg aaaaggtgtc     420
   gacgttcagc tgggccctgt ggccggcccg ctgggcaggt ctcccgatgc tggcagaaac     480
   tgggaaggtt tcgccccgga tcccgtgctg accggaaaca tgatggcgtc caccatccag     540
   ggtattcaag atgctggtgt cattgcttgc gccaagcatt tcatcctcta cgagcaggag     600
   catttccgtc agggcgctca agatggctac gatatctccg acagtatcag tgccaacgct     660
   gatgacaaga ctatgcacga gttgtacttg tggccttttg ccgatgctgt tcgcgctggc     720
   gttggttcaa tcatgtgctc ctacaaccag gtgaacaaca gctacgcctg ctccaacagc     780
   tacaccatga acaagctgct caagagcgaa ttgggcttcc aaggcttcgt catgaccgac     840
   tggggtggcc accacagtgg tgtgggttcc gctctcgctg gtttggacat gtcgatgccc     900
   ggagacattg ccttcgacag tggcacctcc ttctggggca ctaacctcac ggttgccgtg     960
   ctcaatggaa gtgttcctga gtggcgtgtt gatgacatgg ctgtccgtat catgtccgct    1020
   tattacaagg tcggccgcga ccgctacagc gtccccatca actttgactc gtggaccctg    1080
   gatacctatg gtcccgagca ctatgcggtg ggccagggca acaccaagat caacgagcac    1140
   gttgatgttc gcggcaacca tgcagaaatc atccatgaaa tcggtgctgc cagcgccgtc    1200
   cttctcaaga acaagggtgg gcttcctttg actggcaccg aacggtttgt cggtgttttt    1260
   ggagaggatg ccggatccaa cccttggggt gtgaacggct gcagtgaccg aggctgcgac    1320
   aatggtacat tggccatggg ctggggcagt ggtactgcta acttccccta cttggtgacg    1380
   ccggagcagg cgatcgagag agaagtcgtg tcccgaaatg gaaccttcac cgccatcacg    1440
   gacaatggcg ctcttgagca gatggcggct gtcgcctctc aggctgatgt ttgcctggtc    1500
   ttcgccaacg ccgactccgg agaaggctac atcaacgtcg acggcaatga gggtgaccgg 1560
   aagaatctga ccctgtggca aggggcggat caagtcatcc acaacgtcac tgccaactgc 1620
   aacaacaccg tcgtggtgtt gcacactgtc ggccccgttt tgatcgatga ttggtatgac 1680
   caccccaacg tcactgccat tctctgggct ggtcttccgg gccaggagag cggtaactcg 1740
   ctcgtcgatg tcctctacgg ccgtgtcaac cctggcggaa agactccgtt cacctgggga 1800
   cggacccggg aggattacgg tgctcctctg gtcctcaagc cgaacaatgg caagggcgcc 1860
   ccgcagcagg acttcactga gggtatcttc atcgactacc gtcggtttga caagtacaac 1920
   atcaccccca tctacgaatt cggattcggt ctgagctaca ctacctttga gttttctgag 1980
   ctcaatgtgc agcctatcaa tacgccgccg tacactcccg cttctggctt caccaaggcg 2040
   gcgcagtcat tcggcccgtc gtccaatgct tctgacaacc tgtaccccag cgacattgag 2100
   cgggtcccgt tgtacatcta cccatggctc aactccaccg atttgaaggc gtccgccaat 2160
   gaccctgact atgggttgcc taacgacaaa tacgttcctc ccaacgccac gaacggtaac 2220
   ccgcagccca ttaacccggc tggcggtgct cctggtggca accctagtct ctatgagcct 2280
   gttgctcggg tctcagccat catcaccaac accggtaagg ttacgggtga cgaggttcct 2340
   caactgtatg tctctcttgg cggtcccgat gatgctccca aggttcttcg tggcttcgac 2400
   cgtatcacac ttgcgcctgg tcagcagacc ttgtggacga ccaccctgac gaggcgagac 2460
   atctcgaact gggaccctgt cacccagaac tgggttgtga ccaactacac caagacggtg 2520
   tatgttggta actcatcccg caacctgcct ttgcaggcac cccttaagcc ataccctgga 2580
   attgcttctg ctcatcatca ccatcaccac taa 2613
<210> 7
   <211> 12030
   <212> DNA
   <213> Artificial Sequence
<220>
   <223> promotor test palsmid
<400> 7
   ttaccaatgc ttaatcagtg aggcacctat ctcagcgatc tgtctatttc gttcatccat        60
   agttgcctga ctccccgtcg tgtagataac tacgatacgg gagggcttac catctggccc       120
   cagtgctgca atgataccgc gagacccacg ctcaccggct ccagatttat cagcaataaa       180
   ccagccagcc ggaagggccg agcgcagaag tggtcctgca actttatccg cctccatcca       240
   gtctattaat tgttgccggg aagctagagt aagtagttcg ccagttaata gtttgcgcaa       300
   cgttgttgcc attgctacag gcatcgtggt gtcacgctcg tcgtttggta tggcttcatt       360
   cagctccggt tcccaacgat caaggcgagt tacatgatcc cccatgttgt gcaaaaaagc       420
   ggttagctcc ttcggtcctc cgatcgttgt cagaagtaag ttggccgcag tgttatcact      480
   catggttatg gcagcactgc ataattctct tactgtcatg ccatccgtaa gatgcttttc      540
   tgtgactggt gagtactcaa ccaagtcatt ctgagaatag tgtatgcggc gaccgagttg      600
   ctcttgcccg gcgtcaatac gggataatac cgcgccacat agcagaactt taaaagtgct      660
   catcattgga aaacgttctt cggggcgaaa actctcaagg atcttaccgc tgttgagatc      720
   cagttcgatg taacccactc gtgcacccaa ctgatcttct gcatctttta ctttcaccag      780
   cgtttctggg tgagcaaaaa caggaaggca aaatgccgca aaaaagggaa taagggcgac      840
   acggaaatgt tgaatactca tactcttcct ttttcaatat tattgaagca tttatcaggg      900
   ttattgtctc atgagcggat acatatttga atgtatttag aaaaataaac aaataggggt      960
   tccgcgcaca tttccccgaa aagtgccacc tgacgtcatt taaatgttta aaccagacag     1020
   acagtctcga gtgcccagag cggcgccctg caggctaggt tcgatgccga tattccgatt     1080
   tctccacgtt ctgagaggga aagcggcaga catcgtagtt acctgctcga gtgggattac     1140
   ttcacactgc cttgtcttct tggtgaggaa aagacgaggc tggtctcttt ctttctatca     1200
   tcttccataa gtcagaggca atcgcctggg cttgtgatgt cactactggc gcgcatgtta     1260
   tttgcctcaa gtccgccaag tacctcccgt catgtcgcgg tatctcttcc ttgttttccc     1320
   atagtccctc atgaggtcac actggcctct agaattagac tgtatattgc gacttatagt     1380
   gacgatcaag acgaacgagg aagatgcttc tgcttgcata ctcgtgtcta gtccgccata     1440
   ttacaatcag attgactggc gcttgcgact ggaagcctct caggtgattg tatagtagaa     1500
   gtcttgtatc gtatgctcct gatagaggtg atatttcaag tagtaccagc tgatatgctc     1560
   acagacaaac tacctccctg ttgtgaaaag taagttttca caactacaac ctttctgaga     1620
   ggctagcaat agagattcgg acctcccaac tggtgactat agtcaataga caattttcac     1680
   cgcagtagaa aaatccttat catgataaca tacgcagccg tactctcttg acgtccgagt     1740
   tgtcttgtgg agcccagcca tggtcagtat gtaagtccaa aggaagtaca aacaactacc     1800
   tggtgatcat gtttaatcta ggtaaaaata tgcataaatc gacgatcgta gcggcgctag     1860
   tttgcgctca caccacacag cactctctcg cccaatgatg tccggatgtg ggaatactag     1920
   ggtgaaacac cgcccccttc ttgagagagc aacaacaatc attctgctgt cggcagaaga     1980
   gcagagactt gctgacccta gttaatgact acacagctcg gaggtttgtg acatgtccat     2040
   gattttgata catggcggag agcaatgtgg tggacgaaat caatcaccat atggcgctat     2100
   attggctgtt tcaggtcctg tttcaagctg ttcctacagc tctttcttgg tctacttgtg     2160
   gtcgcctgct acatcagttg atatacccgg aattactgca gccacttgca gtcccgtgga     2220
   attctcacgg tgaatgtagg ccttttgtag ggtaggaatt gtcactcaag cacccccaac     2280
   ctccattacg cctcccccat agagttccca atcagtgagt catggcactg ttctcaaata 2340
   gattggggag aagttgactt ccgcccagag ctcaaggtcg cacaaccgca tgatataggg 2400
   tcggcaacgg caaaaaagca cgtggctcac cgaaaagcaa gatgtttgcg atctaacatc 2460
   caggaacctg gatacatcca tcatcacgca cgaccacttt gatctgctgg taaactcgta 2520
   ttcgccctaa accgaagtgc gtggtaaatc tacacgtggg cccctttcgg tatactgcgt 2580
   gtgtcttctc taggtgccat tcttttccct tcctctagtg ttgaattgtt tgtgttggag 2640
   tccgagctgt aactacctct gaatctctgg agaatggtgg actaacgact accgtgcacc 2700
   tgcatcatgt atataatagt gatcctgaga aggggggttt ggagcaatgt gggactttga 2760
   tggtcatcaa acaaagaacg aagacgcctc ttttgcaaag ttttgtttcg gctacggtga 2820
   agaactggat acttgttgtg tcttctgtgt atttttgtgg caacaagagg ccagagacaa 2880
   tctattcaaa caccaagctt gctcttttga gctacaagaa cctgtggggt atatatctag 2940
   agttgtgaag tcggtaatcc cgctgtatag taatacgagt cgcatctaaa tactccgaag 3000
   ctgctgcgaa cccggagaat cgagatgtgc tggaaagctt ctagcgagcg gctaaattag 3060
   catgaaaggc tatgagaaat tctggagacg gcttgttgaa tcatggcgtt ccattcttcg 3120
   acaagcaaag cgttccgtcg cagtagcagg cactcattcc cgaaaaaact cggagattcc 3180
   taagtagcga tggaaccgga ataatataat aggcaataca ttgagttgcc tcgacggttg 3240
   caatgcaggg gtactgagct tggacataac tgttccgtac cccacctctt ctcaaccttt 3300
   ggcgtttccc tgattcagcg tacccgtaca agtcgtaatc actattaacc cagactgacc 3360
   ggacgtgttt tgcccttcat ttggagaaat aatgtcattg cgatgtgtaa tttgcctgct 3420
   tgaccgactg gggctgttcg aagcccgaat gtaggattgt tatccgaact ctgctcgtag 3480
   aggcatgttg tgaatctgtg tcgggcagga cacgcctcga aggttcacgg caagggaaac 3540
   caccgatagc agtgtctagt agcaacctgt aaagccgcaa tgctgaagac tccatcacta 3600
   ctatgtatag gaagttggcc gtcatctcgg ccttcttggc cacagctcgt gcccagagcg 3660
   gcgccctaga gaacctggcg tattcacctc ccttctaccc ttcgccgtgg gccaatggac 3720
   agggcgactg ggcagaggcc tacgagaagg ccgtcaagtt tgtctcccaa ctcacgctgg 3780
   ccgaaaaggt caacctgacc accggaactg gttgggagca ggaccgatgc gtcggccaag 3840
   tgggtagcat cccaagattg ggcttcccag gactttgcat gcaggactct ccgctgggtg 3900
   ttcgagacac tgactacaac tcggccttcc cggcgggtgt caatgtcgct gctacctgga 3960
   acagggacct cgcctaccgt cgcggccaag cgatgggcga ggagcatcgc ggaaaaggtg 4020
   tcgacgttca gctgggccct gtggccggcc cgctgggcag gtctcccgat gctggcagaa 4080
   actgggaagg tttcgccccg gatcccgtgc tgaccggaaa catgatggcg tccaccatcc 4140
   agggtattca agatgctggt gtcattgctt gcgccaagca tttcatcctc tacgagcagg 4200
   agcatttccg tcagggcgct caagatggct acgatatctc cgacagtatc agtgccaacg 4260
   ctgatgacaa gactatgcac gagttgtact tgtggccttt tgccgatgct gttcgcgctg 4320
   gcgttggttc aatcatgtgc tcctacaacc aggtgaacaa cagctacgcc tgctccaaca 4380
   gctacaccat gaacaagctg ctcaagagcg aattgggctt ccaaggcttc gtcatgaccg 4440
   actggggtgg ccaccacagt ggtgtgggtt ccgctctcgc tggtttggac atgtcgatgc 4500
   ccggagacat tgccttcgac agtggcacct ccttctgggg cactaacctc acggttgccg 4560
   tgctcaatgg aagtgttcct gagtggcgtg ttgatgacat ggctgtccgt atcatgtccg 4620
   cttattacaa ggtcggccgc gaccgctaca gcgtccccat caactttgac tcgtggaccc 4680
   tggataccta tggtcccgag cactatgcgg tgggccaggg caacaccaag atcaacgagc 4740
   acgttgatgt tcgcggcaac catgcagaaa tcatccatga aatcggtgct gccagcgccg 4800
   tccttctcaa gaacaagggt gggcttcctt tgactggcac cgaacggttt gtcggtgttt 4860
   ttggagagga tgccggatcc aacccttggg gtgtgaacgg ctgcagtgac cgaggctgcg 4920
   acaatggtac attggccatg ggctggggca gtggtactgc taacttcccc tacttggtga 4980
   cgccggagca ggcgatcgag agagaagtcg tgtcccgaaa tggaaccttc accgccatca 5040
   cggacaatgg cgctcttgag cagatggcgg ctgtcgcctc tcaggctgat gtttgcctgg 5100
   tcttcgccaa cgccgactcc ggagaaggct acatcaacgt cgacggcaat gagggtgacc 5160
   ggaagaatct gaccctgtgg caaggggcgg atcaagtcat ccacaacgtc actgccaact 5220
   gcaacaacac cgtcgtggtg ttgcacactg tcggccccgt tttgatcgat gattggtatg 5280
   accaccccaa cgtcactgcc attctctggg ctggtcttcc gggccaggag agcggtaact 5340
   cgctcgtcga tgtcctctac ggccgtgtca accctggcgg aaagactccg ttcacctggg 5400
   gacggacccg ggaggattac ggtgctcctc tggtcctcaa gccgaacaat ggcaagggcg 5460
   ccccgcagca ggacttcact gagggtatct tcatcgacta ccgtcggttt gacaagtaca 5520
   acatcacccc catctacgaa ttcggattcg gtctgagcta cactaccttt gagttttctg 5580
   agctcaatgt gcagcctatc aatacgccgc cgtacactcc cgcttctggc ttcaccaagg 5640
   cggcgcagtc attcggcccg tcgtccaatg cttctgacaa cctgtacccc agcgacattg 5700
   agcgggtccc gttgtacatc tacccatggc tcaactccac cgatttgaag gcgtccgcca 5760
   atgaccctga ctatgggttg cctaacgaca aatacgttcc tcccaacgcc acgaacggta 5820
   acccgcagcc cattaacccg gctggcggtg ctcctggtgg caaccctagt ctctatgagc 5880
   ctgttgctcg ggtctcagcc atcatcacca acaccggtaa ggttacgggt gacgaggttc 5940
   ctcaactgta tgtctctctt ggcggtcccg atgatgctcc caaggttctt cgtggcttcg 6000
   accgtatcac acttgcgcct ggtcagcaga ccttgtggac gaccaccctg acgaggcgag 6060
   acatctcgaa ctgggaccct gtcacccaga actgggttgt gaccaactac accaagacgg 6120
   tgtatgttgg taactcatcc cgcaacctgc ctttgcaggc accccttaag ccataccctg 6180
   gaattgcttc tgctcatcat caccatcacc actaataagg gctctgcgtc tccaggatcc 6240
   ggcgcgcctc tagaggcttt cgtgaccggg cttcaaacaa tgatgtgcga tggtgtggtt 6300
   cccggttggc ggagtctttg tctactttgg ttgtctgtcg caggtcggta gaccgcaaat 6360
   gagcaactga tggattgttg ccagcgatac tataattcac atggatggtc tttgtcgatc 6420
   agtagctagt gagagagaga gaacatctat ccacaatgtc gagtgtctat tagacatact 6480
   ccgagaataa agtcaactgt gtctgtgatc taaagatcga ttcggcagtc gagtagcgta 6540
   taacaactcc gagtaccagc gaaagcacgt cgtgacagga gcagggcttt gccaactgcg 6600
   caaccttgct tgaatgagga tacacggggt gcaacatggc tgtactgatc catcgcaacc 6660
   aaaatttctg tttatagatc aagctggtag attccaatta ctccacctct tgcgcttctc 6720
   catgacatgt aagtgcacgt ggaaaccata cccaaattgc ctacagctgc ggagcatgag 6780
   cctatggcga tcagtctggt catgttaacc agcctgtgct ctgacgttaa tgcagaatag 6840
   aaagccgcgg ttgcaatgca aatgatgatg cctttgcaga aatggcttgc tcgctgactg 6900
   ataccagtaa caactttgct tggccgtagg gctctgcgtc tccactcgag ataacggtga 6960
   gactagcggc cggtcccctt atcccagctg ttccacgttg gcctgcccct cagttagcgc 7020
   tcaactcaat gcccctcact ggcgaggcga gggcaaggat ggaggggcag catcgcctga 7080
   gttggagcaa agcggccgcc atgggagcag cgaaccaacg gagggatgcc gtgctttgtc 7140
   gtggctgctg tggccaatcc gggcccttgg ttggctcaca gagcgttgct gtgagaccat 7200
   gagctattat tgctaggtac agtatagaga gaggagagag agagagagag agagagaggg 7260
   gaaaaaaggt gaggttgaag tgagaaaaaa aaaaaaaaaa aaaaatccaa ccactgacgg 7320
   ctgccggctc tgccaccccc ctccctccac cccagaccac ctgcacactc agcgcgcagc 7380
   atcacctaat cttggctcgc cttcccgcag ctcaggttgt tttttttttc tctctccctc 7440
   gtcgaagccg cccttgttcc cttatttatt tccctctcca tccttgtctg cctttggtcc 7500
   atctgcccct ttgtctgcat ctcttttgca cgcatcgcct tatcgtcgtc tcttttttca 7560
   ctcacgggag cttgacgaag acctgactcg tgagcctcac ctgctgattt ctctcccccc 7620
   ctcccgaccg gcttgacttt tgtttctcct ccagtacctt atcgcgaagc cggaagaacc 7680
   tcttaacctc tagatgaaaa agcctgaact caccgcgacg tctgtcgaga agttcctgat 7740
   cgaaaagttc gacagcgtct ccgacctgat gcagctctcg gagggcgaag aatctcgtgc 7800
   tttcagcttc gatgtaggag ggcgtggata tgtcctgcgg gtaaatagct gcgccgatgg 7860
   tttctacaaa gatcgttatg tttatcggca ctttgcatcg gccgcgctcc cgattccgga 7920
   agtgcttgac attggggaat tcagcgagag cctgacctat tgcatctccc gccgtgcaca 7980
   gggtgtcacg ttgcaagacc tgcctgaaac cgaactgccc gctgttctgc agccggtcgc 8040
   ggaggccatg gatgcgatcg ctgcggccga tctcagccag acgagcgggt tcggcccatt 8100
   cggaccgcaa ggaatcggtc aatacactac atggcgtgat ttcatatgcg cgattgctga 8160
   tccccatgtg tatcactggc aaactgtgat ggacgacacc gtcagtgcgt ccgtcgcgca 8220
   ggctctcgat gagctgatgc tttgggccga ggactgcccc gaagtccggc acctcgtgca 8280
   cgcggatttc ggctccaaca atgtcctgac ggacaatggc cgcataacag cggtcattga 8340
   ctggagcgag gcgatgttcg gggattccca atacgaggtc gccaacatct tcttctggag 8400
   gccgtggttg gcttgtatgg agcagcagac gcgctacttc gagcggaggc acccggagct 8460
   tgcaggatcg ccgcggctcc gggcgtatat gctccgcatt ggtcttgacc aactctatca 8520
   gagcttggtt gacggcaatt tcgatgatgc agcttgggcg cagggtcgat gcgacgcaat 8580
   cgtccgatcc ggagccggga ctgtcgggcg tacacaaatc gcccgcagaa gcgcggccgt 8640
   ctggaccgat ggctgtgtag aagtactcgc cgatagtgga aaccgacgcc ccagcactcg 8700
   tccgagggca aaggaatagg tttaaacagc tccgtggcga aagcctgacg caccggtaga 8760
   ttcttggtga gcccgtatca tgacggcggc gggagctaca tggccccggg tgatttattt 8820
   tttttgtatc tacttctgac ccttttcaaa tatacggtca actcatcttt cactggagat 8880
   gcggcctgct tggtattgcg atgttgtcag cttggcaaat tgtggctttc gaaaacacaa 8940
   aacgattcct tagtagccat gcattttaag ataacggaat agaagaaaga ggaaattaaa 9000
   aaaaaaaaaa aaacaaacat cccgttcata acccgtagaa tcgccgctct tcgtgtatcc 9060
   cagtaccacg gcaaaggtat ttcatgatcg ttcaatgttg atattgttcc cgccagtatg 9120
   gctccacccc catctccgcg aatctcctct tctcgaacgc ggtagtggcg cgccaattgg 9180
   taatgaccca tagggagaca aacagcataa tagcaacagt ggaaattagt ggcgcaataa 9240
   ttgagaacac agtgagacca tagctggcgg cctggaaagc actgttggag accaacttgt 9300
   ccgttgcgag gccaacttgc attgctgtca agacgatgac aacgtagccg aggaccgtca 9360
   caagggacgc aaagttgtcg cggatgaggt ctccgtagat ggcatagccg gcaatccgag 9420
   agtagcctct caacaggtgg ccttttcgaa accggtaaac cttgttcaga cgtcctagcc 9480
   gcagctcacc gtaccagtat cgaggattga cggcagaata gcagtggctc tccaggattt 9540
   gactggacaa aatcttccag tattcccagg tcacagtgtc tggcagaagt cccttctcgc 9600
   gtgcgagtcg aaagtcgcta tagtgcgcaa tgagagcaca gtaggagaat aggaacccgc 9660
   gagcacattg ttcaatctcc acatgaattg gatgactgct gggcagaatg tgctgcctcc 9720
   aaaatcctgc gtccaacaga tactctggca ggggcttgag atgaatgcct ctgggccccc 9780
   agataagatg cagctctgga ttctcggtta cgatgatatc gcgagagagc acgagttggt 9840
   gatggagggg acgaggaggc ataggtcggc cgcaggccca taaccagtct tgcacagcat 9900
   tgatcttcct cacgaggagc tcctgatgca gaaactcctc catgttgctg attgggttga 9960
   gaatttcatc gctcctggat cgtatggttg ctggcaagac cctgcttaac cgtgccgtgt 10020
   catggtcatc tctggtggct tcgtcgctgg cctgtctttg caattcgaca gcaaatggtg 10080
   gagatctctc tatcgtgaca gtcatggtag cgatagctag gtgtcgttgc acgcacatag 10140
   gccgaaatgc gaagtggaaa gaatttcccg gcgcggaatg aagtctcgtc attttgtact 10200
   cgtactcgac acctccaccg aagtgttaag aatggatcca cgatgccaaa aagcttgttc 10260
   atttcggcta gcccgtgatc ctggcgcttc tagggctgaa actgtgttgt taatgtatta 10320
   ttggctgtgt aactgacttg aatggggaat gaggagcgcg atggattcgc ttgcatgtcc 10380
   cctggccaag acgagccgct ttggcggttt gtgattcgaa ggtgtgtcag cggaggcgcc 10440
   agggcaacac gcactgagcc agccaacatg cattgctgcc gacatgaata gacacgcgcc 10500
   gagcagacat aggagacgtg ttgactgtaa aaattctact gaatattagc acgcatggtc 10560
   tcaataagag caataggaat gcttgccaat cataagtacg tatgtgcttt ttcctgcaaa 10620
   tggtacgtac ggacagttca tgttgtctgt catcccccac tcaggctctc atgatcattt 10680
   tatgggactg gggttttgct gactgaatgg attcagccgc acgaaacaaa ttgggggcca 10740
   tgcagaaggg aagccccccc agccccctgt tcataatttg ttaagagtcg gagagctgcc 10800
   tagtatgaag cagcaattga taacgttgac tttgcgcatg agctctcaag ccgggcatat 10860
   gtatcacgtt tctgcctaga gccgcacggg acccaagaag ctcttgtcat aaggtattta 10920
   tgagtgttca gctgccaacg ctggttctac tttggctcaa ccgcatccca taagctgaac 10980
   tttgggagct gccagaatgt ctcttgatgt acagcgatca acaaccgtgc gccggtcgac 11040
   aactgttcac cgatcaggga cgcgaagagg acccaatccc gcggttaacg cacctgctcc 11100
   gaagaagcaa aagggctatg aggtggtgca gcaaggaatc aaagagctct atccacttga 11160
   caaggccacc tgcaggactg tctctcctct ctagtttaaa catttaaata catgtgagca 11220
   aaaggccagc aaaaggccag gaaccgtaaa aaggccgcgt tgctggcgtt tttccatagg 11280
   ctccgccccc ctgacgagca tcacaaaaat cgacgctcaa gtcagaggtg gcgaaacccg 11340
   acaggactat aaagatacca ggcgtttccc cctggaagct ccctcgtgcg ctctcctgtt 11400
   ccgaccctgc cgcttaccgg atacctgtcc gcctttctcc cttcgggaag cgtggcgctt 11460
   tctcatagct cacgctgtag gtatctcagt tcggtgtagg tcgttcgctc caagctgggc 11520
   tgtgtgcacg aaccccccgt tcagcccgac cgctgcgcct tatccggtaa ctatcgtctt 11580
   gagtccaacc cggtaagaca cgacttatcg ccactggcag cagccactgg taacaggatt 11640
   agcagagcga ggtatgtagg cggtgctaca gagttcttga agtggtggcc taactacggc 11700
   tacactagaa gaacagtatt tggtatctgc gctctgctgt agccagttac cttcggaaaa 11760
   agagttggta gctcttgatc cggcaaacaa accaccgctg gtagcggtgg tttttttgtt    11820
   tgcaagcagc agattacgcg cagaaaaaaa ggatctcaag aagatccttt gatcttttct    11880
   acggggtctg acgctcagtg gaacgaaaac tcacgttaaa gggattttgg tcatgagatt     11940
   atcaaaaagg atcttcacct agatcctttt aaattaaaaa tgaagtttta aatcaatcta    12000
   aagtatatat gagtaaactt ggtctgacag                                     12030